(19) Europäisches Patentamt
European Patent Office
Office européen des brevets

(11) Numéro de publication : **0 677 531 A1**

# (12) DEMANDE DE BREVET EUROPEEN

(21) Numéro de dépôt : **95400776.1**

(22) Date de dépôt : **07.04.95**

(51) Int. Cl.[6] : **C07K 5/065,** A61K 38/05

(30) Priorité : **12.04.94 FR 9404288**

(43) Date de publication de la demande :
**18.10.95 Bulletin 95/42**

(84) Etats contractants désignés :
**AT BE CH DE DK ES FR GB GR IE IT LI LU NL PT SE**

(71) Demandeur : **SYNTHELABO**
**22, Avenue Galilée**
**F-92350 Le Plessis Robinson (FR)**

(72) Inventeur : **Mallart, Sergio**
**17ter avenue du Maréchal Joffre**
**F-91400 Orsay (FR)**
Inventeur : **Lassalle, Gilbert**
**53 avenue Victor Hugo**
**F-92140 Clamart (FR)**
Inventeur : **Purcell, Thomas Andrew**
**47bis rue de la Millière Les Mesnuls**
**F-78490 Montfort l'Amaury (FR)**
Inventeur : **Muller, Jean Claude**
**4 avenue de l'Espérance**
**F-91390 Morsang sur Orge (FR)**

(74) Mandataire : **Thouret-Lemaitre, Elisabeth et al**
**SYNTHELABO,**
**Service Brevets,**
**B.P. 72**
**F-92352 Le Plessis-Robinson Cédex (FR)**

(54) **Dérivés de peptides boroniques, leur préparation et leur application en thérapeutique.**

(57) Composés de formule (I)

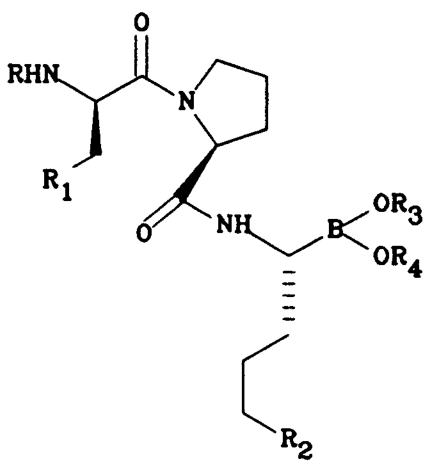

(I)

dans laquelle
R représente soit un atome d'hydrogène, soit un groupe $(C_1-C_4)$alkyle droit ou ramifié, soit un groupe $-CO(C_1-C_4)$alkyle droit ou ramifié, soit un groupe $-CO_2(C_1-C_4)$alkyle droit ou ramifié,
$R_1$ représente soit un groupe phényle, soit un groupe cyclohexyle,
$R_2$ représente soit un groupe

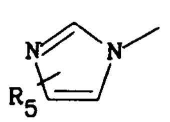
,

soit un groupe

EP 0 677 531 A1

Jouve, 18, rue Saint-Denis, 75001 PARIS

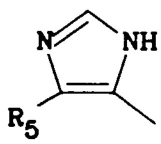 ,

où $R_5$ est un atome d'hydrogène ou un groupe ($C_1$-$C_4$)alkyle et
$R_3$ et $R_4$ soit représentent chacun un atome d'hydrogène, soit représentent ensemble le résidu d'un composé dihydroxylé tel que par exemple le butane-2,3-diol, le 2,3-diméthylbutane-2,3-diol ou le (1α, 3α, 5α)-2,6,6-triméthylbicyclo[3.1.1]heptane-2,3-diol [(+)-α-pinanediol] sous forme d'isomères optiques ou géométriques, purs ou sous forme de mélanges, ainsi que leurs sels d'addition aux acides et aux bases pharmaceutiquement acceptables.
Application en thérapeutique.

La présente invention a pour objet des dérivés de peptides boroniques, leur préparation et leur application en thérapeutique.

Les composés de l'invention répondent à la formule (I)

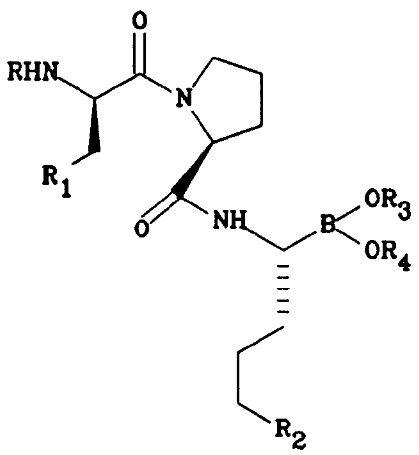

(I)

dans laquelle
R représente soit un atome d'hydrogène, soit un groupe ($C_1$-$C_4$)alkyle droit ou ramifié, soit un groupe -CO($C_1$-$C_4$)alkyle droit ou ramifié, soit un groupe -$CO_2$($C_1$-$C_4$)alkyle droit ou ramifié,
$R_1$ représente soit un groupe phényle, soit un groupe cyclohexyle,
$R_2$ représente soit un groupe

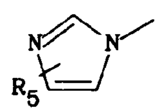

,

soit un groupe

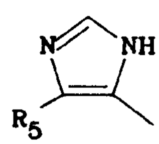

,

où $R_5$ est un atome d'hydrogène ou un groupe ($C_1$-$C_4$)alkyle et
$R_3$ et $R_4$ soit représentent chacun un atome d'hydrogène, soit représentent ensemble le résidu d'un composé dihydroxylé tel que par exemple le butane-2,3-diol, le 2,3-diméthylbutane-2,3-diol ou le (1α, 3α, 5α)-2,6,6-triméthylbicyclo[3.1.1]heptane-2,3-diol [(+)-α-pinanediol].

Suivant la définition des substituants $R_3$ et $R_4$ les composés de l'invention possèdent 3 ou 7 centres asymétriques.
Les composés de l'invention peuvent exister sous forme d'isomères optiques ou géométriques, purs ou sous forme de mélanges qui fon également partie de l'invention
Selon l'invention les composés préférés ayant 3 centres asymétriques sont les dérivés de *D*-alanyl-*L*-prolinamide de configuration [2(*R*)] et les composé préférés ayant 7 centres asymétriques sont les dérivés de *D*-alanyl-*N*-[(4,6-méthano-1, 3,2-benzodioxaborol-2-yl)méthyl]-*L*-prolinamide de configuration [3a*S*,[2(*R*), 3aα, 4β, 6β, 7aα]].

Les composés de l'invention peuvent exister sous forme de bases libres ou de sels d'addition aux acides et aux bases pharmaceutiquement acceptables qui font également partie de l'invention.

Parmi les composés selon l'invention, les composés préférés sont ceux pour lesquels $R_2$ représente un groupe

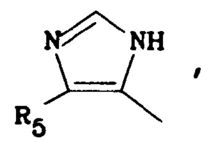

où $R_5$ est un atome d'hydrogène ou un groupe $(C_1-C_4)$alkyle.

Parmi ceux-ci, les composés de choix sont ceux pour lesquels, R représente un atome d'hydrogène ou un groupe $(C_1-C_4)$alkyle droit ou ramifié,

$R_1$ représente un groupe phényle et

$R_3$ et $R_4$ représentent chacun un atome d'hydrogène.

Enfin le composé le plus intéressant est l'acide (*R*)-[1-[(*D*-phénylalanyl-*L*-prolyl)amino]-4-(1*H*-imidazol-4-yl)butyl] boronique et ses sels d'addition aux acides et aux bases pharmaceutiquement acceptables.

Les composés de l'invention pour lesquels $R_2$ représente un groupe

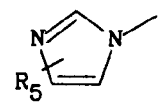

peuvent être synthétisés selon le schéma 1.

On fait réagir un tripeptide boronique de formule (II) (dans laquelle $R_1$ est tel que défini précédemment, $R_3$ et $R_4$

Schéma 1

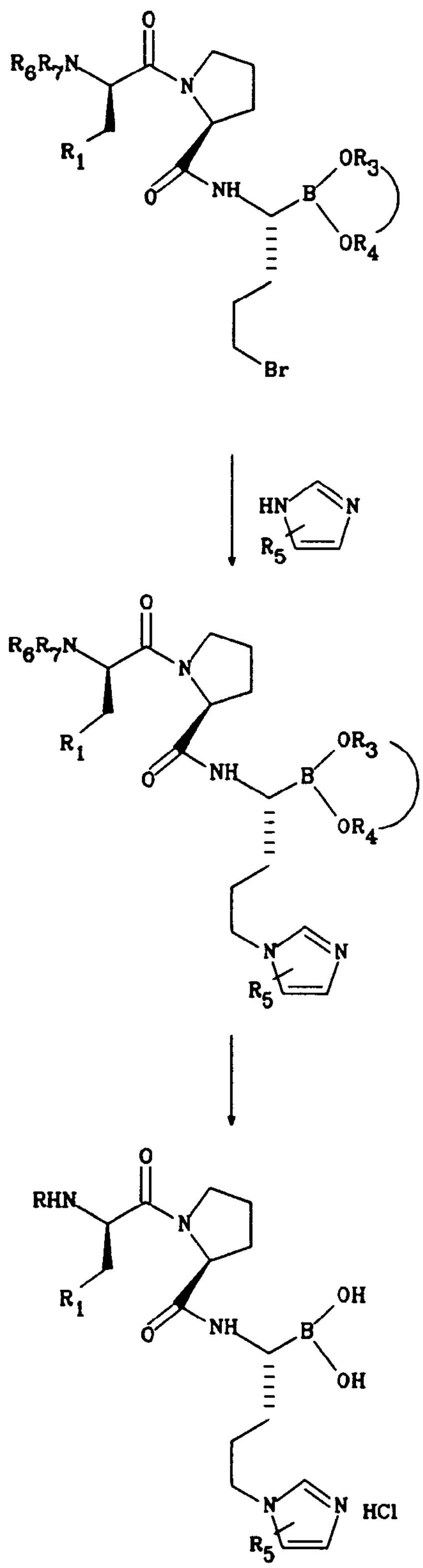

(II)

(III)

(Ia)

(Ib)

représentent ensemble un résidu d'un composé dihydroxylé tel que défini précédemment, $R_6$ représente soit

un atome d'hydrogène quand $R_7$ représente un groupe -CO($C_1$-$C_4$)alkyle droit ou ramifié, soit un groupe -$CO_2$($C_1$-$C_4$)alkyle droit ou ramifié quand $R_7$ représente un atome d'hydrogène ou un groupe ($C_1$-$C_4$)alkyle droit ou ramifié) avec un composé de formule (III) (dans laquelle $R_5$ est un atome d'hydrogène ou un groupe ($C_1$-$C_4$)alkyle), dans un solvant tel que par exemple le dioxane, pour obtenir un composé de formule (Ia). Ensuite, si on désire obtenir un composé de formule (Ib) (dans laquelle R, $R_1$ et $R_5$ sont tels que définis précédemment), on peut faire réagir le composé de formule (Ia) avec de l'acide chlorhydrique ou du trichlorure de bore.

Les composés de l'invention pour lesquels $R_2$ représente un groupe

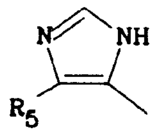

où $R_5$ est un atome d'hydrogène ou un groupe ($C_1$-$C_4$)alkyle, peuvent être synthétisés selon le schéma 2.

On fait réagir le [3*a*S-(3*a*α, 4β, 6β, 7*a*α)]-2-(3-bromopropyl)-3*a*,5,5-triméthylhexahydro-4,6-méthano-1,3,2-benzodioxaborole de formule (IV) avec de l'iodure de sodium dans un solvant tel que par exemple l'acétone pour obtenir le [3*a*S-(3*a*α, 4β, 6β, 7*a*α)]-2-(3-iodopropyl)-3*a*,5,5-triméthylhexahydro-4,6-méthano-1,3,2-benzodioxaborole de formule (V) que l'on condense avec un composé de formule (VI) (dans laquelle $R_5$ est tel que défini précédemment) pour obtenir le composé de formule (VII); la réaction est réalisée dans un solvant tel que le tétrahydrofurane, à une température comprise entre -78 et +20 °C. Ensuite, selon un procédé analogue à celui décrit par Mattheson dans Organomettallics, (1984), 3, 614, on fait réagir le composé de formule (VII) avec du dichlorométhyllithium, en présence de chlorure de zinc, dans un solvant tel que le tétrahydrofurane, à une température comprise entre -100 et +20 °C, pour obtenir le composé de formule (VIII) que l'on fait réagir avec du bis(triméthylsilyl)amidure de

## Schéma 2

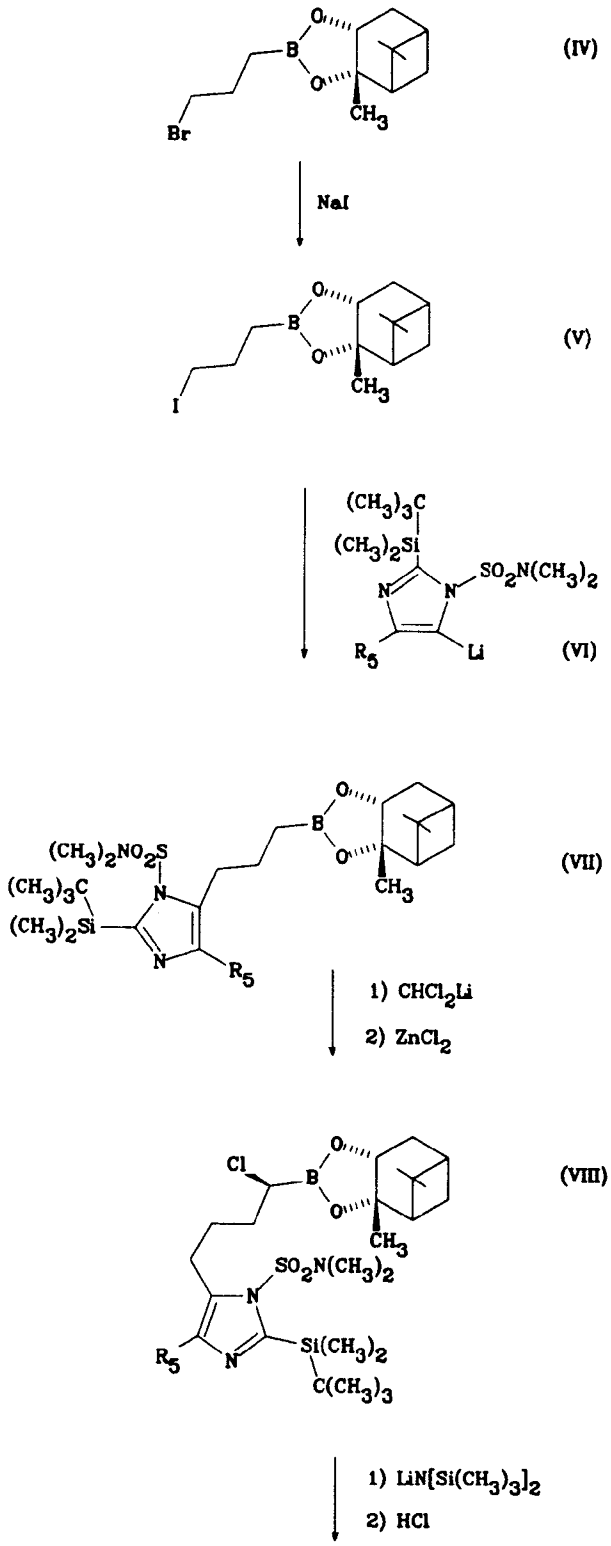


(IV)

(V)

(VI)

(VII)

(VIII)

Schéma 2 (suite)

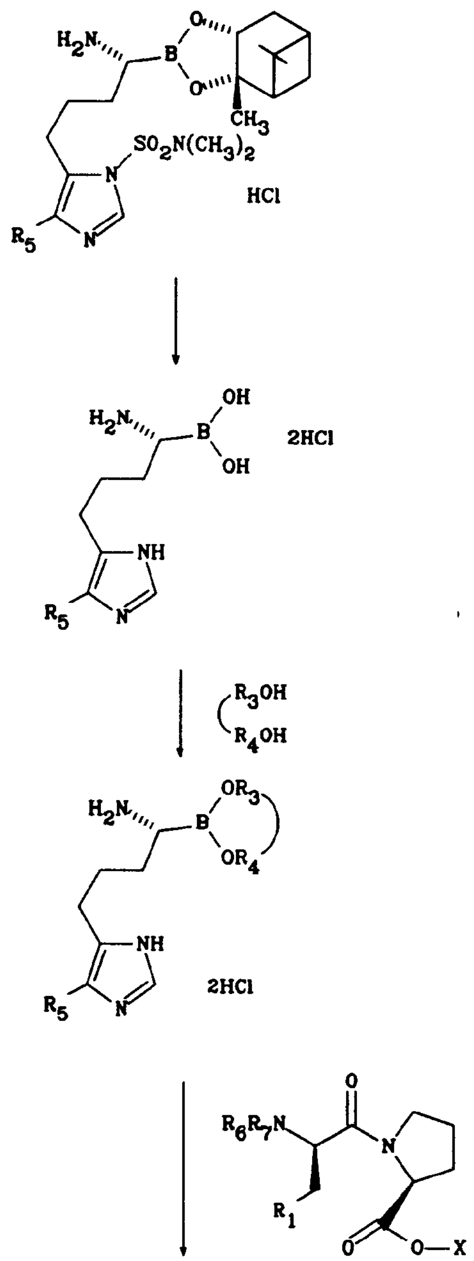


(IX)

(X)

(XI)

(XII)

(XIII)

## Schéma 2 (suite)

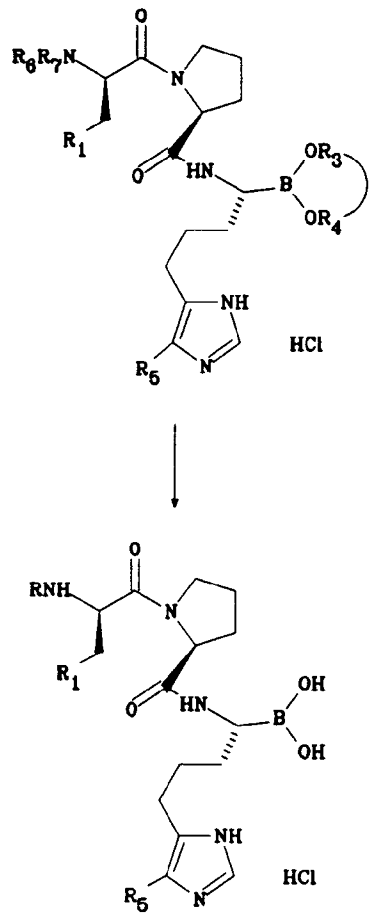

(Ic)

(Id)

lithium ; la réaction est réalisée dans un solvant tel que le tétrahydrofurane à une température comprise entre -78 et +20 °C. On obtient un composé que l'on traite avec de l'acide chlorhydrique dans un solvant tel que le dioxane et on obtient le chlorhydrate de formule (IX) que l'on hydrolyse pour obtenir le dichlorhydrate de l'acide (R)-α-aminobutaneboronique de formule (X) que l'on fait réagir avec un diol de formule (XI) (dans laquelle $R_3$ et $R_4$ représentent ensemble le résidu d'un composé dihydroxylé tel que défini précédemment) pour obtenir le chlorhydrate de formule (XII) ; ensuite on condense le composé de formule (XII) avec une forme activée d'un dipeptide de formule (XIII) (dans laquelle $R_1$ est tel que défini précédemment, $R_6$ représente soit un atome d'hydrogène quand $R_7$ représente un groupe $-CO(C_1-C_4)$alkyle droit ou ramifié, soit un groupe $-CO_2(C_1-C_4)$alkyle droit ou ramifié quand $R_7$ représente un atome d'hydrogène ou un groupe $(C_1-C_4)$alkyle droit ou ramifié et X représente soit le groupe pyrrolidin-1-yl-2,5-dione, soit le groupe 2-méthylpropyloxycarbonyle) pour obtenir un composé que l'on traite par de l'acide chlorhydrique et on obtient le composé de formule (Ic). Ensuite, si on désire obtenir un composé de formule (Id) (dans laquelle R, $R_1$ et $R_5$ sont tels que définis précédemment), on peut faire réagir le composé de formule (Ic) avec de l'acide chlorhydrique ou du trichlorure de bore.

Les composés de départ sont disponibles dans le commerce ou décrits dans la littérature ou peuvent être préparés selon des méthodes qui y sont décrites ou qui sont connues de l'homme du métier.

Ainsi des tripeptides de formule (II) sont décrits dans la demande de brevet européen no 0293881.

La *N*-acétyl-*D*-phénylalanyl-*L*-proline, le [3a*S*-[2(*R*), 3aα, 4β, 6β,7aα)]-*N*-acétyl-*D*-phénylalanyl-*N*-[4-bromo-1-(3a,5,5-triméthylhexahydro-4,6-méthano-1,3,2-benzodioxaborol-2-yl)butyl]-*L*-prolinamide et la 1-[*N*-[(1,1-diméthyléthoxy]-*D*-phénylalanyl]-*L*-proline sont décrits par Kettner et coll. dans J. Biol. Chem. (1990), <u>265</u>, 18289.

Le [3a*S*-(3aα, 4β, 6β, 7aα)]-2-(3-bromopropyl)-3a,5,5-triméthylhexahydro-4,6-méthano-1,3,2-benzodioxaborole est décrit dans le brevet européen no 0293881.

Le 2-[(1,1-diméthyléthyl)diméthylsilyl]-*N*,*N*-diméthyl-1*H*-imidazole-l-sulfonamide est décrit par Ngochindo dans J. Chem. Soc. Perkin Trans. (1990), 1, 1645.
La préparation des composés de formule (XII) est décrite dans la demande de brevet français FR 9404287.
La préparation des composés de formule (XIII) (dans laquelle $R_6$ représente un groupe $-CO_2(C_1-C_4)$alkyle droit ou ramifié et $R_7$ représente un groupe $(C_1-C_4)$alkyle droit ou ramifié) est réalisée à partir des composés correspondants de formule (XIII) (dans laquelle $R_7$ représente un atome d'hydrogène) selon une méthode analogue à celle décrite par Bajusz dans J. Med. Chem., (1990), 33, 1729-1735.

Les exemples suivants illustrent la préparation de certains composés conformément à l'invention.
Les microanalyses élémentaires et les spectres IR et RMN confirment la structure des composés obtenus.
Les numéros des composés exemplifiés renvoient à ceux du tableau donné plus loin.
Le rapport entre parenthèse est le rapport molaire acide:base.

Exemple 1 (composé 1)

chlorhydrate de [3a*S*-[2(*R*), 3aα, 4β, 6β, 7aα]]-*N*-acétyl-*D*-phénylalanyl-*N*-[4-(1*H*-imidazol-1-yl)-1-(3*a*,5,5-triméthylhexahydro-4,6-méthano-1,3,2-benzodioxaborol-2-yl)butyl]-*L*-prolinamide (1:1)

1.1. [3a*S*-[2(*R*), 3aα, 4β, 6β, 7aα]]-*N*-acétyl-*D*-phénylalanyl-*N*-[4-(1*H*-imidazol-1-yl)-1-(3*a*,5,5-triméthylhexahydro-4,6-méthano-1,3,2-benzodioxaborol-2-yl)butyl)-*L*-prolinamide

On dissout 3 g (4,8 mmoles) de [3a*S*-[2(*R*), 3aα, 4β, 6β,7aα]]-*N*-acétyl-*D*-phénylalanyl-*N*-[4-bromo-1-(3*a*,5,5-triméthylhexahydro-4,6-méthano-1,3,2-benzodioxaborol-2-yl)butyl]-*L*-prolinamide et 0,902 g (9,6 mmoles) de 1*H*-imidazole dans 10 ml de dioxane. On chauffe à 80 °C pendant 6 heures puis on évapore le solvant sous vide. On reprend le résidu dans 50 ml de dichlorométhane puis on lave la phase organique successivement avec une solution aqueuse d'hydrogénocarbonate de sodium à 5 % et avec une solution saturée en chlorure de sodium. On sèche sur sulfate de sodium, on filtre et on évapore. On purifie par chromatographie sur colonne de gel de silice en éluant par un mélange méthanol:dichlorométhane (5:95).
On obtient 1,2 g de produit sous forme d'une huile.
Rendement = 41 %
$[\alpha]_D^{20}$ = -77,5 ° (c = 1,5; chloroforme)

1.2. chlorhydrate de [3a*S*-[2(*R*), 3aα, 4β, 6β, 7aα]]-*N*-acétyl-*D*-phénylalanyl-*N*-[4-(1*H*-imidazol-1-yl)-1-(3*a*,5,5-triméthylhexahydro-4,6-méthano-1,3,2-benzodioxaborol-2-yl)butyl]-*L*-prolinamide (1:1)

On solubilise dans 3 ml de chloroforme, 1,2 g (1,97 mmoles) de [3a*S*-[2(*R*), 3aα, 4β, 6β, 7aα]]-*N*-acétyl-*D*-phénylalanyl-*N*-[4-(1*H*-imidazol-1-yl)-1-(3*a*,5,5-triméthyl-hexahydro-4,6-méthano-1,3,2-benzodioxaborol-2-yl)butyl]-*L*-prolinamide et on refroidit la solution à 0 °C. On ajoute 20 ml d'une solution d'acide chlorhydrique 0,1 N dans l'isopropanol, on agite pendant 15 minutes à 0 °C et on concentre sous vide. On sèche sur pentoxyde de phosphore et on triture le résidu dans l'éther.
On obtient 1 g de produit sous forme d'un solide amorphe.
Point de fusion = 99 °C  Rendement = 78 %
$[\alpha]_D^{20}$ = -90,4 ° (c = 1,1; chloroforme)

Exemple 2 (composé 2)

chlorhydrate de (*R*)-*N*-acétyl-*D*-phénylalanyl-*N*-[1-borono-4-(1*H*-imidazol-1-yl)butyl]-*L*-prolinamide (1:1)

On solubilise dans 6 ml de dichlorométhane anhydre, 700 mg (1,2 mmoles) de chlorhydrate de [3a*S*-[2(*R*), 3aα, 4β, 6β, 7aα]]-*N*-acétyl-*D*-phénylalanyl-*N*-[4-(1*H*-imidazol-1-yl)-1-(3*a*,5,5-triméthylhexahydro-4,6-méthano-1,3,2-benzodioxaborol-2-yl)butyl]-*L*-prolinamide. On refroidit la solution à -78 °C et on ajoute goutte à goutte, en 15 minutes, 5 ml (5 mmoles) d'une solution 1 M de trichlorure de bore dans le dichlorométhane. On agite pendant 15 minutes à -78 °C et pendant 45 minutes à 0 °C, puis on ajoute lentement 15 ml d'eau. On agite la solution pendant 30 minutes à 0 °C puis on ajoute une solution d'acide acétique à 10 %. On sépare les phases, on extrait la phase aqueuse avec 3 fois 10 ml d'éther et on évapore à sec. On purifie le résidu par chromatographie sur colonne Biogel P2® en éluant par de l'acide acétique à 10 %. On concentre les fractions contenant le produit et on triture le résidu dans l'éther. On obtient un produit sous forme d'une poudre blanche que l'on dilue dans 2 ml d'eau. On obtient une solution que l'on purifie par chromatographie sur colonne Biorad AG 1W8 ®(forme OH) en éluant par de l'eau et par une solution 1 N d'acide chlorhydrique. On concentre les fractions contenant le produit et on triture le résidu dans l'éther.
On obtient 50 mg de produit.
Point de fusion = 168-175 °C (d)  Rendement = 10 %

$[\alpha]_D^{20}$ = -118,5 ° (c = 0,6; eau)

Exemple 3 (composé 3)

chlorhydrate de [3a*S*-[2(*R*), 3aα, 4β, 6β, 7aα]]-*N*-acétyl-*D*-phénylalanyl-*N*-[4-(1*H*-imidazol-4(5)-yl)-1-(3*a*,5,5-triméthylhexahydro-4,6-méthano-1,3,2-benzodioxaborol-2-yl)butyl]-*L*-prolinamide (1:1)

3.1. chlorhydrate de [3a*S*-[2(*R*), 3aα, 4β, 6β, 7aα]]-α-(3*a*,5,5-triméthylhexahydro-4,6-méthano-1,3,2-benzodioxaborol-2-yl)-1*H*-imidazole-4(5)-butanamine (2:1)

3.1.1. [3a*S*-(3aα, 4β, 6β, 7aα)]-2-(3-iodopropyl)-3*a*,5,5-triméthylhexahydro-4,6-méthano-1,3,2-benzodioxaborole

On porte à la température de reflux pendant 24 heures, une solution de 37 g (122 mmoles) de [3a*S*-(3aα, 4β, 6β, 7aα)]-2-(3-bromopropyl)-3*a*,5,5-triméthylhexahydro-4,6-méthano-1,3,2-benzodioxaborole et de 72,7 g (488 mmoles) d'iodure de sodium dans 500 ml d'acétone. On évapore le solvant et on reprend le résidu dans un mélange de 500 ml d'éther et de 100 ml d'eau contenant 1 g de sulfite de sodium. On sèche la phase organique sur du sulfate de magnésium, on filtre et on évapore.
On obtient 40 g de produit que l'on utilise directement dans l'étape suivante.
Rendement = 95 %

3.1.2. [3a*S*-(3aα, 4β, 6β, 7aα)]-2-[(1,1-diméthyléthyl)diméthylsilyl]-*N*,*N*-diméthyl-4(5)-[3-(3*a*,5,5-triméthylhexahydro-4,6-méthano-1,3,2-benzodioxaborol-2-yl)propyl]-1*H*-imidazole-1-sulfonamide

On dissout 70,5 g (244 mmoles) de 2-[(1,1-diméthyléthyl)diméthylsilyl]-*N*,*N*-diméthyl-1*H*-imidazole-1-sulfonamide dans 250 ml de tétrahydrofurane. On refroidit le milieu réactionnel à -78 °C et on ajoute 152 ml (244 mmoles) d'une solution 1,6 M de n-butyllithium dans l'hexane. On laisse sous agitation pendant 1 heure à -78 °C puis on ajoute 40 g (115 mmoles) de [3a*S*-(3aα, 4β, 6β, 7aα)]-2-(3-iodopropyl)-3*a*,5,5-triméthylhexahydro-4,6-méthano-1,3,2-benzodioxaborole en solution dans 100 ml de tétrahydrofurane. On agite le milieu réactionnel entre -78 °C et +20 °C pendant 1 heure puis à 20 °C pendant 2 heures. On verse le milieu réactionnel sur 350 ml d'un mélange glace-eau contenant 14,5 g (121 mmoles) d'hydrogénosulfate de sodium. On extrait la phase aqueuse par 3 fois 100 ml d'éther, on réunit les phases éthérées, on les sèche sur sulfate de magnésium, on filtre et on évapore. On purifie le résidu par chromatographie sur colonne de gel de silice en éluant par une solution d'acétate d'éthyle à 20 % dans l'hexane.
On obtient 45 g de produit.
Rendement = 73 %
$[\alpha]_D^{20}$ = +12,5 ° (c = 1,9; chloroforme)

3.1.3. [3a*S*-[2(*S*), 3aα, 4β, 6β, 7aα]]-4(5)-[4-chloro-4-(3*a*,5,5-triméthylhexahydro-4,6-méthano-1,3,2-benzodioxaborol-2-yl)butyl]-2-[(1,1-diméthyléthyl)diméthylsilyl]-*N*,*N*-diméthyl-1*H*-imidazole-1-sulfonamide

On refroidit une solution de 8,3 g (98 mmoles) de dichlorométhane dans 100 ml de tétrahydrofurane à -100 °C. On ajoute 39,1 ml (98 mmoles) d'une solution 2,5 M de n-butyllithium dans l'hexane. On laisse pendant 15 minutes à cette température puis on ajoute 45 g (89 mmoles) de [3a*S*-(3aα, 4β, 6β, 7aα)]-2-[(1,1-diméthyléthyl)diméthylsilyl]-*N*,*N*-diméthyl-4(5)-[3-(3*a*,5,5-triméthylhexahydro-4,6-méthano-1,3,2-benzodioxaborol-2-yl)propyl]-1*H*-imidazole-1-sulfonamide, en solution dans 50 ml de tétrahydrofurane. On laisse le mélange réactionnel pendant 15 minutes à -100 °C et on ajoute 9,8 g (70 mmoles) d'une solution de chlorure de zinc dans 50 ml de tétrahydrofurane. On laisse revenir à +20 °C pendant 16 heures. On évapore sous vide et on reprend le résidu dans un mélange de 200 ml de dichlorométhane et de 50 ml d'eau. On sépare les phases et on extrait la phase aqueuse avec 100 ml de dichlorométhane. On rassemble les phases organiques, on les sèche sur du sulfate de magnésium, on les filtre et on évapore. On purifie le résidu coloré obtenu, par chromatographie sur colonne de gel de silice en éluant par un mélange acétate d'éthyle:hexane (20:80).
On obtient 40 g de produit sous forme d'une huile incolore.
Rendement = 80 %
$[\alpha]_D^{20}$ = +15,9 ° (c = 2,65; chloroforme)

3.1.4. chlorhydrate de [3a*S*-[2(*R*), 3aα, 4β, 6β, 7aα]]-4(5)-[4-amino-4-(3*a*,5,5-triméthylhexahydro-4,6-méthano-1,3,2-benzodioxaborol-2-yl)butyl]-*N*,*N*-diméthyl-1*H*-imidazole-1-sulfonamide (1:1)

On met en solution 12,6 g (78 mmoles) de 1,1,1,3,3,3-hexaméthyldisilazane, dans 80 ml de tétrahydrofurane et on ajoute 31 ml (78 mmoles) d'une solution 2,5 M de n-butyllithium dans l'hexane. On laisse pendant 1 heure à -78 °C et on ajoute 40 g (71 mmoles) de [3a*S*-[2(*S*), 3aα, 4β, 6β, 7aα]]-4(5)-[4-chloro-4-(3*a*,5,5-triméthylhexahydro-4,6-méthano-1,3,2-benzodioxaborol-2-yl)butyl]-2-[(1,1-diméthyléthyl)diméthylsilyl]-*N*,*N*-diméthyl-1*H*-imidazole-1-sulfonamide, en solution dans 80 ml de tétrahydrofurane. On laisse sous agitation pendant 1 heure à -78 °C et pendant 16 heures à +20 °C. On refroidit le milieu réactionnel à -78 °C, on ajoute 78 ml (312 mmoles) d'une solution 4 N d'acide chlorhydrique dans le dioxane et on laisse sous agitation, pendant 1 heure à -78 °C et pendant 2 heures à +20 °C. On évapore sous vide et on reprend le résidu dans 200 ml de chloroforme. On filtre et on évapore.

On obtient 32 g de produit sous forme d'une huile que l'on triture dans l'éther.

On obtient le produit sous forme d'un solide.

Rendement = 89 %

Point de fusion = 90-92 °C

$[\alpha]_D^{20}$ = +11 ° (c = 1 ; méthanol)

3.1.5. chlorhydrate de [3a*S*-[2(*R*), 3aα, 4β, 6β, 7aα]]-α-(3a,5,5-triméthylhexahydro-4,6-méthano-1,3,2-benzodioxaborol-2-yl)-1*H*-imidazole-4(5)-butanamine (2:1)

On porte au reflux pendant 3 heures, 32 g (70 mmoles) de chlorhydrate de [3a*S*-[2(*R*), 3aα, 4β, 6β, 7aα]]-4(5)-[4-amino-4-(3a,5,5-triméthylhexahydro-4,6-méthano-1,3,2-benzodioxaborol-2-yl)butyl]-*N*,*N*-diméthyl-1*H*-imidazole-1-sulfonamide, en solution dans 200 ml d'acide chlorhydrique 4 N. On extrait la solution par 4 fois 100 ml d'éther et on évapore à sec. On reprend le résidu par 100 ml de méthanol et on ajoute 11,9 g (70 mmoles) de [1*R*-(1α, 2α, 3α, 5α)]-2,6,6-triméthylbicyclo-[3.1.1]heptane-2,3-diol. On laisse sous agitation pendant 16 heures à 20 °C et on évapore à sec.

On obtient 27 g de produit sous forme d'une huile que l'on triture dans l'éther.

On obtient le produit sous forme d'un solide.

Point de fusion = 75-80 °C

3.2. ester de la *N*-acétyl-*D*-phénylalanyl-*L*-proline avec la 1-hydroxypyrrolidine-2,5-dione

On suspend 6 g (20 mmoles) de *N*-acétyl-*D*-phénylalanyl-*L*-proline dans un mélange de 100 ml d'acétate d'éthyle et 5 ml de diméthylformamide. On ajoute 2,53 g (22 mmoles) de 1-hydroxypyrrolidine-2,5-dione et on refroidit à 0 °C. On ajoute ensuite, par petites portions, 4,53 g (22 mmoles) de 1,3-dicyclohexylcarbodiimide sous forme solide. On agite pendant 20 heures à 20 °C et on filtre la suspension. On lave successivement le filtrat avec 20 ml d'une solution d'hydrogénocarbonate de sodium à 5 % puis avec une solution saturée en chlorure de sodium et on sèche sur sulfate de magnésium. On triture le résidu obtenu dans l'éther.

On obtient 8 g de produit vitreux que l'on utilise tel quel dans l'étape suivante.

3.3. chlorhydrate de [3a*S*-[2(*R*), 3aα, 4β, 6β, 7aα]]-*N*-acétyl-*D*-phénylalanyl-*N*-[4-(1*H*-imidazol-4(5)-yl)-1-(3a,5,5-triméthylhexahydro-4,6-méthano-1,3,2-benzodioxaborol-2-yl)butyl]-*L*-prolinamide (1:1)

On solubilise 2,4 g (6,4 mmoles) de chlorhydrate de [3a*S*-[2(*R*), 3aα, 4β, 6β, 7aα]]-α-(3a,5,5-triméthylhexahydro-4,6-méthano-1,3,2-benzodioxaborol-2-yl)-1*H*-imidazole-4(5)-butanamine dans 20 ml de dichlorométhane, on ajoute 2,5 g (6,4 mmoles) de l'ester de la *N*-acétyl-*D*-phénylalanyl-*L*-proline avec la 1-hydroxypyrrolidine-2,5-dione et on refroidit le mélange à -30 °C. On ajoute, goutte à goutte, 3,6 ml (25,6 mmoles) de triéthylamine et on agite pendant 2 heures entre -30 °C et +20 °C puis pendant 2 heures à +20 °C. On ajoute ensuite 20 ml d'une solution aqueuse d'hydrogénocarbonate de sodium à 5 % puis on extrait la phase aqueuse avec 2 fois 20 ml de dichlorométhane. On rassemble les phases organiques, on les sèche sur sulfate de sodium, on les filtre et on évapore.

On reprend le résidu dans 10 ml d'isopropanol et on le traite à 0 °C par 64 ml d'une solution 0,1 N d'acide chlorhydrique dans l'isopropanol. Après évaporation, on décolore le résidu avec du noir animal dans l'acétate d'éthyle et on le purifie sur colonne de Sephadex® LH-20 en éluant par du méthanol. On évapore et on triture le résidu dans l'éther.

On obtient 2 g de produit sous forme d'un solide incolore.

Point de fusion = 130-135 °C Rendement = 51 %

$[\alpha]_D^{20}$ = -112,1 ° (c = 1; chloroforme)

<u>Exemple 4</u> (composé 4)

chlorhydrate de (*R*)-*N*-acétyl-*D*-phénylalanyl-*N*-[1-borono-4-(1*H*-imidazol-4(5)-yl)butyl]-*L*-prolinamide (1:1)

On met en solution 2 g (3 mmoles) de chlorhydrate de (3a*S*-[2(*R*), 3aα, 4β, 6β, 7aα]]-*N*-acétyl-*D*-phénylalanyl-*N*-[4-(1*H*-imidazol-4(5)-yl)-1-(3a,5,5-triméthylhexahydro-4,6-méthano-1,3,2-benzodioxaborol-2-yl)butyl]-*L*-prolinamide, dans 30 ml de dichlorométhane. On refroidit la solution à -78 °C et on ajoute goutte à goutte 12 ml d'une solution 1 M de trichlorure de bore dans le dichlorométhane. On laisse le mélange pendant 15 minutes à -78 °C puis on le place dans un bain de glace et on agite pendant 45 minutes à 0 °C. On ajoute 30 ml d'eau au milieu réactionnel et on sépare les phases. On extrait la phase organique avec 2 fois 25 ml d'une

solution d'acide acétique à 10 % et la phase aqueuse avec 3 fois 25 ml d'éther. On rassemble les phases aqueuses, on les concentre, on dilue le résidu dans 10 ml de méthanol et on évapore. On purifie le résidu par chromatographie sur colonne Bio-gel®P2 en éluant par une solution d'acide acétique à 10 % puis par chromatographie sur colonne Bio-gel®P2 en éluant par une solution 1 mM d'acide chlorhydrique.
On obtient 800 mg de produit.
Point de fusion = 155-160 °C Rendement = 52 %
$[\alpha]_D^{20}$ = -98,5 ° (c = 0,65; eau)

Exemple 5 (composé 5)

chlorhydrate de [3*aS*-(2[*R*[*S*(*R*)]], 3aα, 4β, 6β, 7aα]]-[2-[2-[[[4-(1*H*-imidazol-4-yl)-1-(3*a*,5,5-triméthylhexahydro-4,6-méthano-1,3,2,-benzodioxaborol-2-yl)butyl]amino]carbonyl]pyrrolidin-1-yl]-2-oxo-1-(phénylméthyl) éthyl]carbamate de 1,1-diméthyléthyle (1:1)

On traite à la température ambiante, 7,2 g (7,2 mmoles) de *N*-[(1,1-diméthyléthoxy)carbonyl]-*D*-phénylalanyl-*L*-proline en solution dans 30 ml de tétrahydrofurane avec 2,3 ml (21 mmoles) de N-méthylmorpholine. On fait refroidir le milieu réactionnel à -20 °C et on ajoute 2,72 ml (21 mmoles) d'isobutylchloroformate. On laisse le mélange sous agitation pendant 15 minutes à -20 °C, on ajoute 7,5 g (20 mmoles) de chlorhydrate de [3*aS*-[2(*R*), 3aα, 4β, 6β, 7aα]]-α-(3*a*,5,5-triméthylhexahydro-4,6-méthano-1,3,2-benzodioxaborol-2-yl)-1*H*-imidazole-4(5)-butanamine en solution dans 10 ml de chloroforme et 5,6 ml (40 mmoles) de triéthylamine. On laisse le mélange sous agitation pendant 30 minutes à -20 °C puis 24 heures à la température ambiante. On le dilue par 300 ml d'acétate d'éthyle et on le lave successivement par 200 ml d'eau et 200 ml d'une solution saturée en chlorure de sodium. On le sèche sur sulfate de sodium, on évapore le solvant à sec et on reprend le résidu par 50 ml de dichlorométhane. On prépare le chlorhydrate en ajoutant 20 ml d'une solution d'isopropanol dans l'acide chlorhydrique 0,1 N et on le purifie sur colonne Lichroprep® RP 18 en éluant par un gradient d'acétonitrile dans l'acide chlorhydrique 0,02 N (20 % à 100 %).
On obtient 5 g de produit sous forme de chlorhydrate.
Point de fusion = 115-120 °C Rendement = 45 %
$[\alpha]_D^{20}$ = - 106 ° (c = 1; chloroforme)

Exemple 6 (composé 6)

chlorhydrate de l'acide (*R*)-[1-[(*D*-phénylalanyl-*L*-prolyl)a-mino]-4-(1*H*-imidazol-4-yl)butyl]boronique (3:1)

A partir de 698 mg (1 mmole) de chlorhydrate de [3*aS*-[2[*R*[*S*(*R*)]], 3aα, 4β, 6β, 7aα]]-[2-[2-[[[4-(1*H*-imidazol-4-yl)-1-(3*a*,5,5-triméthylhexahydro-4,6-méthano-1,3,2,-benzodioxaborol-2-yl)butyl]amino]carbonyl]pyrrolidin-1-yl]-2-oxo-1-(phénylméthyl)éthyl]carbamate de 1,1-diméthyléthyle, selon le mode opératoire décrit dans l'exemple 4 et après purification sur colonne Lichroprep® RP 18, en éluant par un gradient d'acétonitrile et d'acide chlorhydrique 0,02 N, on obtient 300 mg du composé attendu.
Point de fusion = 194 °C
$[\alpha]_D^{20}$ = - 131 ° (c = 1,3 ; eau)

Exemple 7 (composé 7)

chlorhydrate de [3*aS*-[2[*R*[*S*(*R*)]], 3aα, 4β, 6β, 7aα]]-[2-[2-[[[4-(5-méthyl-1*H*-imidazol-4-yl)-1-(3*a*,5,5-triméthylhexahydro-4,6-méthano-1,3,2,-benzodioxaborol-2-yl)butyl]amino] carbonyl]pyrrolidin-1-yl]-2-oxo-1-(phénylméthyl)éthyl] carbamate de 1,1-diméthyléthyle (1:1)

On le prépare selon la méthode décrite dans l'exemple 5 à partir du chlorhydrate de la [3*aS*-[2(*R*), 3aα, 4β, 6β, 7aα]]-5-méthyl-α-(3*a*,5,5-triméthylhexahydro-4,6-méthano-1,3,2-benzodioxaborol-2-yl)-1*H*-imidazole-4-butanamine.
On obtient 600 mg de produit.
Point de fusion = 85-90 °C
$[\alpha]_D^{20}$ = - 53,2 (c = 0,86 ; méthanol)

Le tableau suivant illustre la structure chimique et les propriétés chimiques de quelques composés selon l'invention. Dans la colonne "$R_1$" $-C_6H_5$ représente le groupe phényle et $-C_6H_{11}$ le groupe cyclohexyle.
Dans la colonne "Sel", "HCl" représente un chlorhydrate et le rapport entre parenthèses représente le rapport molaire acide:base.
Dans la colonne "F (°C)", "(d)" signifie "fusion avec décomposition"

Tableau

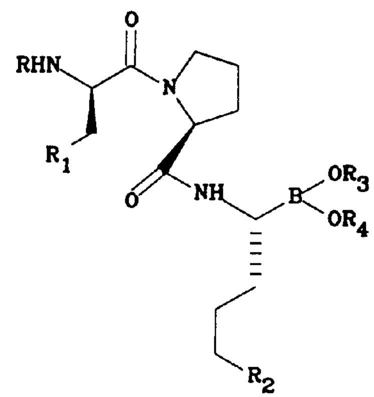

(I)

| No | R | $R_1$ | $R_2$ | $R_3$ | $R_4$ | Sel | $[\alpha]_D^{20}$ (°) (c; solvant) | F (°C) |
|---|---|---|---|---|---|---|---|---|
| 1 | $-COCH_3$ | $-C_6H_5$ | 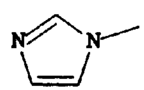 | 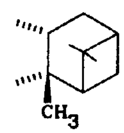 | | HCl (1:1) | -90,4 (1,1; chloroforme) | 99 |
| 2 | $-COCH_3$ | $-C_6H_5$ | 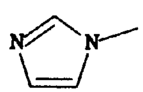 | -H | -H | HCl (1:1) | -118,5 (0,6; eau) | 168-175 (d) |

| No | R | $R_1$ | $R_2$ | $R_3$ | $R_4$ | Sel | $[\alpha]_D^{20}$ (°) (c; solvant) | F (°C) |
|---|---|---|---|---|---|---|---|---|
| 3 | $-COCH_3$ | $-C_6H_5$ | 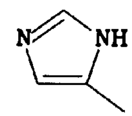 | 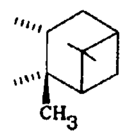 | | HCl (1:1) | -112,1 (1; chloroforme) | 130-135 |
| 4 | $-COCH_3$ | $-C_6H_5$ | 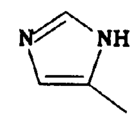 | -H | -H | HCl (1:1) | -98,5 (0,65; eau) | 155-160 |
| 5 | $-COOC(CH_3)_3$ | $-C_6H_5$ | 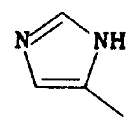 | 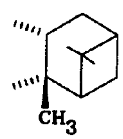 | | HCl (1:1) | -106 (1; chloroforme) | 115-120 |
| 6 | -H | $-C_6H_5$ | 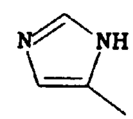 | -H | -H | HCl (3:1) | -131 (1,3; eau) | 194 |
| 7 | $-COOC(CH_3)_3$ | $-C_6H_5$ | 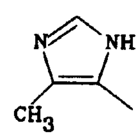 | 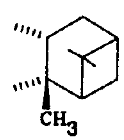 | | HCl (1:1) | -53,2 (0,86 ; méthanol) | 85-90 |

| No | R | $R_1$ | $R_2$ | $R_3$ | $R_4$ | Sel | $[\alpha]_D^{20}$ (°) (c; solvant) | F (°C) |
|---|---|---|---|---|---|---|---|---|
| 8 | -H | $-C_6H_5$ | 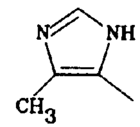 | -H | -H | HCl (2:1) | -129 (0,4 ; eau) | 195-197 |
| 9 | $-COOC(CH_3)_3$ | $-C_6H_{11}$ | 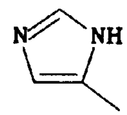 | 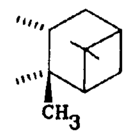 | | HCl (1:1) | -31,2 (0,8 ; méthanol) | 105-110 |
| 10 | -H | $-C_6H_{11}$ | 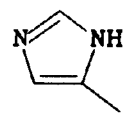 | -H | -H | HCl (2:1) | -84,5 (0,46 ; eau) | 199-201 |
| 11 | $-CH_3$ | $-C_6H_5$ | 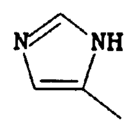 | -H | -H | HCl (2:1) | -131 (1,3 ; eau) | 212-215 |
| 12 | $-CH_3$ | $-C_6H_5$ | 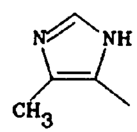 | -H | -H | HCl (2:1) | -104 (0,55 ; eau) | 190-195 |

Les composés de l'invention ont été testés vis-à-vis de la thrombine et de la trypsine *in-vitro* dans les tests suivants.

1. Précipitation du fibrinogène humain par la thrombine bovine.

On incube le composé à tester ou son véhicule (10 µl), pendant 2 minutes, à 37°C, dans une solution de fibrinogène humain (200 µl, 2 mg/ml dans du sérum physiologique). Ensuite, on ajoute 200 µl de thrombine bovine dissous dans de l'eau distillée. La concentration finale de la thrombine est de 0,5 unités NIH/ml. On agite et on note le temps de formation, exprimé en secondes, d'un réseau de fibrine visible. L'inhibition de la fibrinoformation est quantifiée par le calcul de la concentration de composé qui augmente le temps de précipitation de 100 % ($CA_{100}$).

2. Précipitation du fibrinogène humain par la thrombine humaine.

On incube le composé à tester ou son véhicule (10 µl), pendant 2 minutes, à 37°C, dans une solution de fibrinogène humain (200 µl, 2 mg/ml dans du sérum physiologique). Ensuite, on ajoute 200 µl de thrombine humaine dissous dans de l'eau distillée. La concentration finale de la thrombine est de 2 unités NIH/ml. On agite et on note le temps de formation, exprimé en secondes, d'un réseau de fibrine visible. L'inhibition de la fibrinoformation est quantifiée par le calcul de la concentration de composé qui augmente le temps de précipitation de 100 % ($CA_{100}$).

3. Coagulation du plasma de rat par la thrombine bovine.

On anesthésie des rats CD mâles, pesant 150 à 200 g, avec du nembutal (60 mg/kg, 0,1 ml/kg). On prélève le sang sur du citrate trisodique à 3,8 % (1 vol/9 vol de sang) à partir du sinus rétro-orbital. On prépare le plasma par centrifugation à 3600 g pendant 15 minutes, à la température ambiante. On incube le composé à tester ou son véhicule (10 µl) avec 200 µl de plasma, à 37°C, pendant 2 minutes, avant l'addition de 200 µl d'une solution de thrombine bovine. La concentration finale en thrombine est de 0,75 unités NIH/ml. On note le temps de coagulation, exprimé en secondes.
L'inhibition de la thrombine est quantifiée par le calcul de la concentration qui augmente le temps de coagulation de 100% ($CA_{100}$).

4. Agrégation des plaquettes de lapin induite par la thrombine humaine.

On prélève le sang par ponction cardiaque sur du citrate trisodique à 3,8 % (1 vol/9 vol de sang). On le centrifuge à 250 g pendant 10 minutes. On prélève le plasma riche en plaquettes ($P_3P$) ainsi obtenu et on réalise la numération des plaquettes.
Au $P_3P$, on ajoute 2 ng/ml de prostacycline en solution dans du tampon tris à pH 9,0 glacé. On centrifuge à 110 g, pendant 10 minutes et on décante. On ajoute à nouveau de la prostacycline, en solution dans de l'hydroxyde de sodium 50 mM à pH 12, de manière à avoir une concentration finale de 200 ng/ml. On centrifuge à nouveau le $P_3P$ à 800 g pendant 10 minutes. On élimine le plasma pauvre en plaquettes et on met le culot en suspension dans un volume de tyrode contenant 200 ng/ml de prostacycline, volume égal au volume initial de $P_3P$. On centrifuge cette suspension à 800 g pendant 10 minutes. On recommence une seconde fois et dans les mêmes conditions, la mise en suspension du culot et la centrifugation. On remet le culot final en suspension dans une solution de tyrode sans prostacycline et on laisse au repos pendant 2 heures pour permettre l'élimination complète de la prostacycline. On induit l'agrégation de ces plaquettes avec de la thrombine humaine à la concentration finale de 0,3 unités NIH/ml. On enregistre les variations de densité optique au moyen d'un agrégomètre à 4 canaux. On ajoute le composé à tester ou son véhicule à la suspension de plaquettes (volume maximal ajouté de 3 µl), 2 minutes avant l'addition de thrombine. On détermine la concentration qui inhibe l'agrégation de 50 % ($CI_{50}$).

5. Activité vis-à-vis de la trypsine bovine

On incube le composé à tester ou son véhicule (50 µl), pendant 5 minutes, à la température ambiante, avec 50 µl de trypsine bovine dissous dans du tampon tris HCl à pH 8,0. La concentration finale en trypsine est de 229 unités/ml. On déclenche la réaction par l'addition du substrat, la *N* α-benzoyl-L-arginine-4 nitroaniline (50 µl, concentration finale 50 µM). On incube pendant 20 minutes à la température ambiante et on mesure la densité optique de la 4-nitro-aniline libérée, à 405 nm. On calcule la concentration de 4-nitro-aniline à partir d'une courbe d'étalonnage, après avoir soustrait la densité optique des "blancs" (100 µl de tampon + 50 µl de substrat). On détermine la concentration qui inhibe de 50 % l'activité enzymatique ($CI_{50}$).

Parallèlement les composés de l'invention ont été testés vis-à-vis de la coagulation du plasma de rat *ex-*

*vivo.*

On traite les animaux avec le composé à tester ou avec son véhicule, par voie i.v., par voie orale ou par voie sous-cutanée, avant de prélever le sang. On mesure le temps de thrombine comme décrit en 3.

Les composés de l'invention sont des inhibiteurs de la thrombine avec des $CA_{100}$ et des $CI_{50}$ comprises entre $10^{-8}$ et $10^{-6}$ M. Ils ne présentent pas ou présentent peu d'activité inhibitrice vis-à-vis de la trypsine bovine, ce qui démontre leur spécificité.

Ils inhibent la coagulation du plasma de rat à des doses inférieures à 1 mg/kg i.v. et sont également actifs par voie orale et par voie sous cutanée.

Les composés de l'invention peuvent être utiles dans toutes les indications cliniques liées à la thrombose ou dans celles où des complications thrombotiques pourraient intervenir.

A cet effet ils peuvent être présentés sous toutes formes appropriées à l'administration orale, parentérale ou intraveineuse, telles que comprimés, dragées, gélules, capsules, suspensions ou solutions buvables ou injectables, etc. en association avec des excipients convenables, et dosées pour permettre une administration de 1 à 1000 mg par jour et par patient, en une ou plusieurs doses.

## Revendications

1. Composés de formule (I)

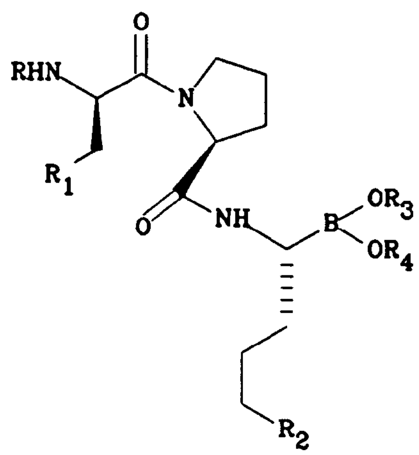

(I)

dans laquelle

R représente soit un atome d'hydrogène, soit un groupe $(C_1-C_4)$alkyle droit ou ramifié, soit un groupe $-CO(C_1-C_4)$alkyle droit ou ramifié, soit un groupe $-CO_2(C_1-C_4)$alkyle droit ou ramifié,

$R_1$ représente soit un groupe phényle, soit un groupe cyclohexyle,

$R_2$ représente soit un groupe

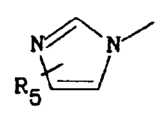

,

soit un groupe

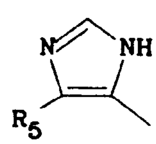

,

où $R_5$ est un atome d'hydrogène ou un groupe $(C_1-C_4)$alkyle et

$R_3$ et $R_4$ soit représentent chacun un atome d'hydrogène, soit représentent ensemble le résidu d'un composé dihydroxylé.

**2.** Composés selon la revendication 1 caractérisés en ce que le composé dihydroxylé est choisi parmi le butane-2,3-diol, le 2,3-diméthylbutane-2,3-diol ou le (1α, 3α, 5α)-2,6,6-triméthylbicyclo[3.1.1]heptane-2,3-diol [(+)-α-pinanediol], sous forme d'isomères optiques ou géométriques, purs ou sous forme de mélanges,
ainsi que leurs sels d'addition aux acides et aux bases pharmaceutiquement acceptables.

**3.** Composés selon la revendication 1 ou 2 caractérisés en ce que la configuration est [2(*R*)) ou [3*aS*,[2(*R*), 3*a*α, 4β, 6β, 7*a*α]] suivant les définitions de $R_3$ et $R_4$.

**4.** Composés selon l'une quelconque des revendications 1 à 3 caractérisés en ce que $R_2$ représente un groupe

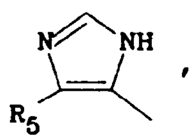

où $R_5$ est un atome d'hydrogène ou un groupe $(C_1\text{-}C_4)$alkyle.

**5.** Composés selon la revendication 4 caractérisés en ce que R représente un atome d'hydrogène ou un groupe $(C_1\text{-}C_4)$alkyle droit ou ramifié, $R_1$ représente un groupe phényle et $R_3$ et $R_4$ représentent chacun un atome d'hydrogène.

**6.** L' acide (*R*)-[1-[(*D*-phénylalanyl-*L*-prolyl)amino]-4-(1*H*-imidazol-4-yl)butyl] boronique ainsi que ses sels d'addition aux acides et aux bases pharmaceutiquement acceptables.

**7.** Procédé de préparation des composés de formule (I) selon la revendication 1 pour lesquels $R_2$ représente un groupe

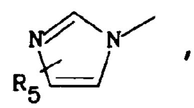

où $R_5$ est un atome d'hydrogène ou un groupe $(C_1\text{-}C_4)$alkyle, procédé caractérisé en ce qu'on fait réagir un tripeptide boronique de formule (II)

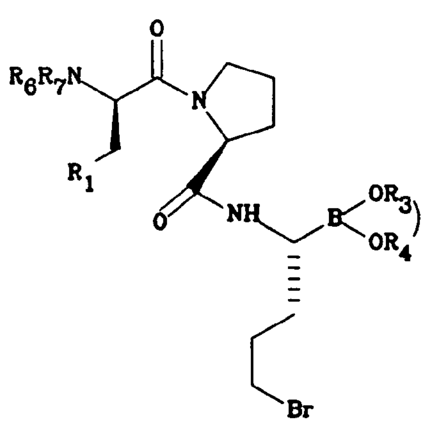

(II)

(dans laquelle $R_1$ est tel que défini dans la revendication 1, $R_3$ et $R_4$ représentent ensemble un résidu d'un composé dihydroxylé tel que défini dans la revendication 1, $R_6$ représente soit un atome d'hydrogène quand $R_7$ représente un groupe $-CO(C_1\text{-}C_4)$alkyle droit ou ramifié, soit un groupe $-CO_2(C_1\text{-}C_4)$alkyle droit ou ramifié quand $R_7$ représente un atome d'hydrogène ou un groupe $(C_1\text{-}C_4)$alkyle droit ou ramifié) avec le composé de formule (III)

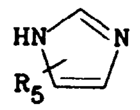

(III)

(dans laquelle $R_5$ est tel que défini dans la revendication 1), pour obtenir un composé de formule (Ia)

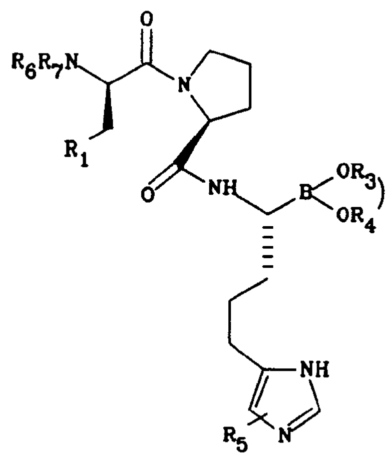

(Ia)

puis si on désire préparer un composé de formule (Ib)

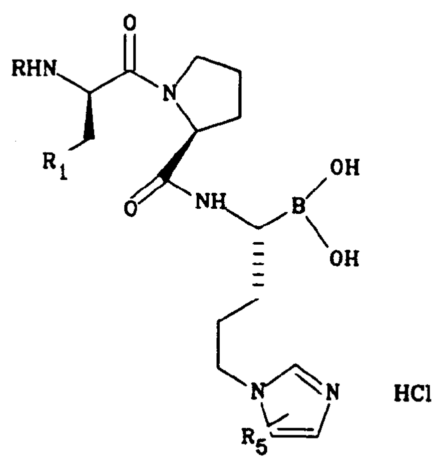

(Ib)

(dans laquelle R, $R_1$ et $R_5$ sont tels que définis dans la revendication 1), on fait réagir le composé de formule (Ia) avec de l'acide chlorhydrique ou du trichlorure de bore.

8. Procédé de préparation des composés de formule (I) selon la revendication 1 pour lesquels $R_2$ représente un groupe

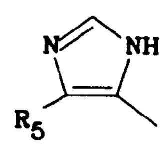

où $R_5$ est un atome d'hydrogène ou un groupe $(C_1$-$C_4)$alkyle, procédé caractérisé en ce que l'on condense le chlorhydrate de formule (XII)

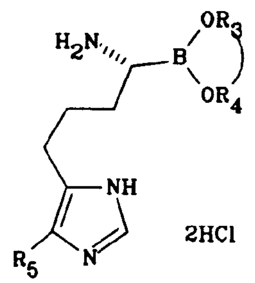

(XII)

(dans laquelle $R_3$ et $R_4$ représentent ensemble un résidu d'un composé dihydroxylé tel que défini dans la revendication 1 et $R_5$ est tel que défini dans la revendication 1) avec une forme activée d'un dipeptide de formule (XIII)

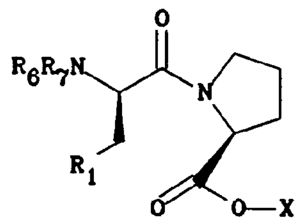

(XIII)

(dans laquelle $R_1$ est tel que défini dans la revendication 1, $R_5$ représente soit un atome d'hydrogène quand $R_7$ représente un groupe -CO(C1-$C_4$)alkyle droit ou ramifié, soit un groupe -$CO_2$($C_1$-$C_4$)alkyle droit ou ramifié quand $R_7$ représente un atome d'hydrogène ou un groupe ($C_1$-$C_4$)alkyle droit ou ramifié et X représente soit le groupe pyrrolidin-1-yl-2,5-dione, soit le groupe 2-méthyl-propyloxycarbonyle), pour obtenir un composé que l'on traite par de l'acide chlorhydrique pour former le composé de formule (Ic)

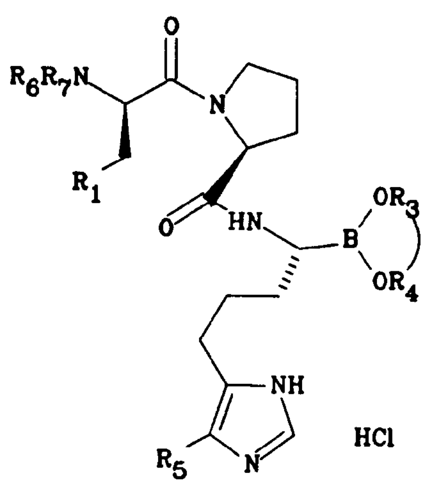

(Ic)

puis si on désire préparer un composé de formule (Id)

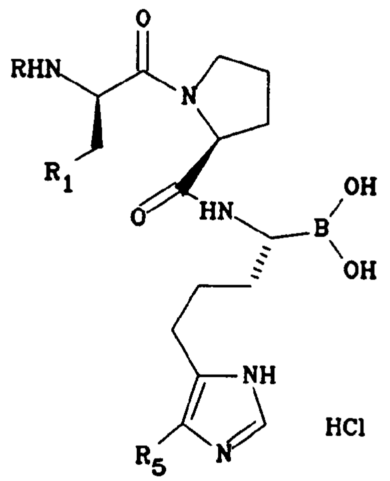

(Id)

(dans laquelle R, $R_1$ et $R_5$ sont tels que définis dans la revendication 1), on fait réagir le composé de formule (Ic) avec de l'acide chlorhydrique ou du trichlorure de bore.

**9.** Médicament, caractérisé en ce qu'il contient un composé selon l'une quelconque des revendications 1 à 6.

**10.** Composition pharmaceutique, caractérisée en ce qu'elle contient un composé selon l'une quelconque des revendications 1 à 6, en association avec tout excipient approprié.

Office européen des brevets

# RAPPORT DE RECHERCHE EUROPEENNE

Numero de la demande
EP 95 40 0776

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | EP-A-0 315 574 (HOECHST AKTIENGESELLSCHAFT)<br>* page 8, ligne 28 - ligne 43; revendications *<br>--- | 1,9,10 | C07K5/065<br>A61K38/05 |
| D,A | EP-A-0 293 881 (E.I DU PONT DE NEMOURS AND COMPANY)<br>* revendications; exemples 22,23,72-110 *<br>--- | 1,9,10 | |
| A | EP-A-0 145 441 (E.I. DU PONT DE NEMOURS AND COMPANY)<br>* revendications; exemples *<br>----- | 1,9,10 | |

DOMAINES TECHNIQUES RECHERCHES (Int.Cl.6)

C07K
A61K

Le présent rapport a été établi pour toutes les revendications

| Lieu de la recherche | Date d'achèvement de la recherche | Examinateur |
|---|---|---|
| LA HAYE | 22 Juin 1995 | Fuhr, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)